# EUROPEAN PATENT SPECIFICATION

(11) **EP 0 642 494 B1**
(45) Date of publication and mention of the grant of the patent: **19.03.1997**
(21) Application number: 93912812.0
(22) Date of filing: 26.05.1993
(51) Int. Cl.: C07C 303/24, C07C 303/44, C11D 1/12

(54) **PROCESS FOR THE PREPARATION OF A SURFACTANT COMPOSITION COMPRISING A SECONDARY ALKYL SULFATE**
VERFAHREN ZUR HERSTELLUNG VON EINEM GRENZFLÄCHENAKTIVEN MITTEL, DAS EIN SEKUNDÄRALKYLSULFAT ENTHÄLT
PROCEDE DE PREPARATION D'UNE COMPOSITION TENSIO-ACTIVE COMPRENANT UN SULFATE D'ALKYLE SECONDAIRE

(30) Priority: 28.05.1992 US 890056; 28.09.1992 US 951955; 12.11.1992 US 974658; 24.11.1992 US 980887; 21.12.1992 US 994022
(43) Date of publication of application: 15.03.1995
(73) Proprietor: SHELL INTERNATIONALE RESEARCH MAATSCHAPPIJ B.V., 2596 HR Den Haag (NL)
(72) Inventor: LUTZ, Eugene, Frederick, Houston, TX 77042 (US); SCHISLA, David, Karl 15035 Westpark Drive, Houston, TX 77082 (US); TOMASKOVIC, Robert, Stephen, Houston, TX 77083 (US); HOEWING, Timothy, Dee, Richmond, TX 77469 (US)
(86) International application number: EP9301339
(87) International publication number: WO9324452

(56) References cited:
- GB-A- 726 994
- GB-A- 965 435
- US-A- 4 175 092

## Description

This invention relates to a process for preparing a surfactant composition comprising a secondary alkyl sulfate, and the surfactant composition so prepared.

In conventional practice, secondary alkyl sulfates have been prepared by reaction of olefins or alcohols with sulfuric acid followed by neutralization of the intermediate secondary alkyl sulfuric acid with aqueous base, usually sodium hydroxide. The process is complicated by incomplete reaction of the starting olefin or alcohol and by formation of dialkyl sulfates which saponify during the neutralization step, noted above, to equal molar amounts of secondary alkyl sulfate and secondary alcohol.

Unreacted olefin and secondary alcohol, which can amount to 50 percent by weight (%wt) or more of the starting olefin, are generally removed from the secondary alkyl sulfate by a process of extraction with an organic solvent as described in U.S. Patent 4,175,092. The extraction process can be complicated by the formation of highly viscous, undesirable emulsions and gels as well as by the dissolution of some of the extracting solvent in the aqueous secondary alkyl sulfate phase. Extracting solvents frequently have objectionable odours and must be removed from the aqueous surfactant solution, an operation which can be accompanied by severe foaming difficulties. When extraction is complete, the concentration of secondary alkyl sulfate in water is generally in the range of 20-40% by weight (F. Asinger, Mono-Olefins: Chemistry and Technology, 1968, pp. 689-694).

It would therefore be advantageous to have a solid and/or non-solid surfactant composition comprising a secondary alkyl sulfate which is substantially free of water and unreacted organic matter, thus allowing maximum flexibility.

A process for preparing surfactant compositions has been found in which secondary alkyl sulfates derived from olefins and/or alcohols can be generated in a manner such that a solid secondary alkyl sulfate-containing product and/or a highly concentrated non-solid secondary alkyl sulfate-containing product are obtained. These products can be used as surfactants and/or detergent compositions and are particularly suitable for household applications.

The invention provides a process for preparing solid and/or non-solid surfactant compositions comprising a secondary alkyl sulfate which are substantially free of unreacted organic matter and water. These surfactant compositions are prepared by the process of claim 1.

Specifically, the present process comprises the steps of: a) sulfating a C₈ to C₂₂ olefin, alcohol and/or mixture thereof (so called detergent range reactants), b) neutralizing the sulfation product of step a) with a base in aqueous solution, c) saponifying the product of step b), wherein in the process, a nonionic organic liquid diluent is added at some point prior to step d) and/or step f) as described in claim 1. In the foregoing process, the nonionic organic liquid diluent can be added during the sulfation step, following the sulfation step, or during the neutralization or saponification steps, provided the diluent is substantially inert in the particular step in which it is added. It is not critical at what point the nonionic organic liquid diluent is added as long as the diluent is present prior to crystallization and/or the phase separation step.

As used herein, the phrase "substantially free of unreacted organic matter and water" refers to compositions which contain less than 10 %wt, preferably less than 5 %wt, of unreacted organic matter and less than 5 %wt, preferably less than 2 %wt, of water.

In step a) of the present process olefins, alcohols or a mixture thereof can be sulfated. In a preferred embodiment, the alkyl sulfuric acid of step a) is prepared by the sulfation of olefins or by the sulfation of a mixture of olefins and alcohols.

British patent specification No. 965,435 discloses substantially water-free crystalline compositions comprising about 90-95 % of a sodium secondary (C8 to C18) alkyl sulphate.

From British patent specification No. 965,435, US patent No. 4,175,092 and British patent specification No. 726,994 processes for the preparation of surfactant compositions comprising secondary alkyl sulphate are known. In such conventional processes the aqueous anionic detergent product obtained after sulphating and neutralizing the olefins or alcohols is separated from the unreacted olefin and alcohol by extraction with organic solvents. The extraction process can be complicated by the formation of highly viscous, undesirable emulsions and gels as well by the dissolution of some of the extracting solvent in the aqueous phase. Further extracting solvents frequently have objectionable odours and must be removed from the aqueous surfactant solution, an operation which can be accompanied by severe foaming difficulties.

When mixtures of olefins and alcohols are used in the preparation of the surfactant composition, the mixture suitably comprises from 40% to 90% olefins and from 10% to 60% alcohols. A preferred mixture includes from 60% to 80% olefins and from 20% to 40% alcohols.

The olefins which are suitably used in the present process can be alpha olefins or internal olefins and they may be linear or branched, but are preferably linear or lightly branched. Single cut olefins or mixtures of olefins may also be used. In a particularly preferred embodiment, the olefin contains from 12 to 18 carbon atoms.

The alcohols which are suitable for use in the preparation contain from 8 to 22 carbon atoms. Acyclic aliphatic alcohols having from 9 to 18 carbon atoms form a preferred class of reactants, particularly the secondary alcohols, as secondary alcohols make up a portion of the potential recycle stream in the present process, although primary alcohols can also be used. When primary alcohols are used in combination with olefins and/or secondary alcohols, the product obtained will be a mixture of primary alkyl sulfates and secondary alkyl sulfates. As a general rule, the alcohols may be of branched or straight chain structure, although alcohol reactants in which greater than 50%, more preferably greater than 60%, and most preferably greater than 70% of the molecules are of linear (straight-chain) carbon structure are preferred.

The sulfating agent used in step a) is a compound capable of forming the carbon to oxygen to sulfur bonds necessary for the formation of an alkyl sulfate. The choice of a particular known sulfating agent typically depends on the compounds to be sulfated. Examples of suitable sulfating agents include sulfuric acid or sulfuric acid salts for the sulfation of olefins, and sulfur trioxide, chlorosulfonic acid or oleum for the sulfation of alcohols. In a preferred embodiment, the olefin or the mixture of olefin and alcohol is sulfated with concentrated sulfuric acid, typically in an amount of from 75%wt to 100%wt, preferably from 85 %wt to 98 %wt, in water. Suitable amounts of sulfuric acid are generally in the range of from 0.3 moles to 1.3 moles of sulfuric acid per mole of olefin and/or alcohol, preferably from 0.4 moles to 1.0 mole of sulfuric acid per mole of olefin and/or alcohol.

The sulfation reaction which results in a solution containing the alkyl sulfuric acid is typically carried out at temperatures in the range of from -20 °C to 50 °C, preferably from 5 °C to 40 °C, and at pressures in the range of from 101 to 507 kPa (1 to 5 atm), preferably from 101 to 203 kPa (1 to 2 atm), and more preferably about 101 kPa (1 atm). Suitable residence times for the sulfation reaction range from a few minutes to several hours, preferably from 2 minutes to 10 hours and more preferably, from 5 minutes to 3 hours.

The sulfation reaction for a suitable C₈ to C₂₂ olefin may be illustrated by the following equation: 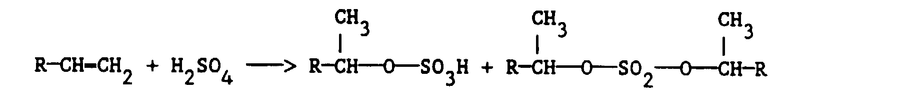 wherein R is an alkyl group having from 6 to 20 carbon atoms. The sulfation reaction results in a solution containing both the alkyl sulfuric acid and the dialkyl sulfate.

In one embodiment, this solution may, prior to the contact with an aqueous solution of a base in step b) or prior to neutralization, be subjected to de-acidification for the partial or substantially complete removal of any unreacted sulfuric acid or any other unreacted sulfating agent. Suitable de-acidification procedures include washing the sulfation reaction product with water.

The solution obtained in step a) is contacted in step b) with an aqueous solution of a base in order to neutralize the alkyl sulfuric acid portion thereof to form the corresponding sulfuric acid salts. The neutralization reaction is accomplished using an aqueous solution of one or more bases such as ammonium or alkali metal or alkaline earth metal hydroxides or carbonates or bicarbonates. Suitable bases include sodium hydroxide, sodium carbonate, potassium hydroxide, calcium hydroxide and the like, with sodium hydroxide or potassium hydroxide being the preferred base. The concentration of the aqueous solution of base is suitably from 10 %wt to 85 %wt base, preferably from 15 %wt to 75 %wt, and more preferably from 20 %wt to 50 %wt, in water. In a preferred embodiment, the base used is sodium hydroxide or potassium hydroxide, and the concentration of the aqueous solution of base is from 10 %wt to 75 %wt base, preferably from 20 %wt to 50 %wt, in water. Generally, an amount of base in excess of the amount required to neutralize the alkyl sulfuric acids and saponify the dialkyl sulfates is used. Suitable amounts of base are generally in the range of from 1.2 moles to 1 mole of base per equivalent of sulfuric acid, alkyl sulfuric acid and dialkyl sulfate, and preferably in the range of from 1.1 moles to 1.0 mole of base.

The neutralization procedure can be carried out over a wide range of temperatures and pressures. Typically, the neutralization procedure is carried out at a temperature in the range of from 20 °C to 65 °C, and a pressure in the range of from 101 to 203 kPa (1 to 2 atm). The neutralization time is typically in the range of from 0.2 hours to 1.0 hours.

If desired, the product may be de-salted following the neutralization reaction. A de-salting treatment may be used in place of or in addition to the de-acidification described above, depending on the extent of the de-acidification. De-salting is typically carried out by using an excess of base in the neutralization reaction which neutralizes the unreacted sulfuric acid to form the inorganic salts thereof in addition to neutralizing the secondary alkyl sulfuric acids. For example, sodium sulfate may be present when sodium hydroxide is the base in the neutralization. These inorganic salts may be removed as a separate phase by known methods such as, for example, filtration. However, removal of the inorganic salts in this manner results in a loss of sulfuric acid, since the organic salts thereof are normally discarded. For this reason, removal of unreacted sulfuric acid by de-acidification via water washing following sulfation is preferred.

Following the contact in step b) of the alkyl sulfuric acid-containing solution with an aqueous solution of a base to effect neutralization, the product of step b), is heated in step c) to a temperature in the range of from 70 °C to 115 °C, preferably from 80 °C to 105 °C in order to effect saponification or hydrolysis of the dialkyl sulfates to form alkyl sulfuric acid salts and secondary alcohols and remove substantially all of the water from the product of step b). In this step, water may be azeotropically distilled to remove a liquid containing a water phase which is discarded. This step is typically carried out using a Dean Stark trap or other similar device. Alternatively, water may be removed along with glycol from the lower phase obtained following the phase separation step (step f).

The saponification procedure can be carried out over a wide range of temperature and pressures. The saponification procedure is typically carried out at a temperature in the range of from 80 °C to 105 °C and a pressure of from 101 to 203 kPa (1 to 2 atm). The saponification reaction is generally carried out over a time period ranging from 0.25 hours to 5.0 hours.

The neutralization and saponification reactions may be illustrated by the following equations: 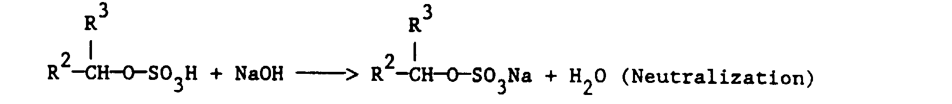 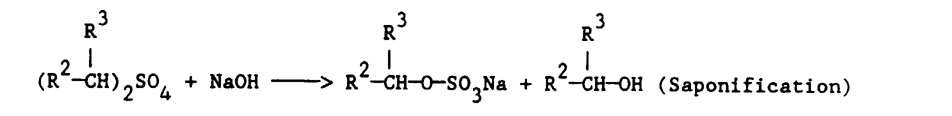 wherein R² and R³ are alkyl groups having in total from 7 to 21 carbon atoms.

Following saponification and removal of substantially all of the water, the resulting product, in accordance with step d) is then cooled to a temperature in the range of from 20 °C to 85 °C to effect crystallization of the solid secondary alkyl sulfate from the saponified product of step c). The cooling step generally takes place over a period of 0.25 hours to 18 hours, preferably 0.5 hours to 16 hours, although both shorter and longer time periods are also acceptable. During the cooling step, the solubility of secondary alkyl sulfate is further reduced and additional secondary alkyl sulfate solids are formed.

Alternatively (second embodiment), following saponification and removal of substantially all of the water, the resulting mixture from step c) is contacted and mixed in step f) with a glycol in the presence of a nonionic organic liquid diluent.

The nonionic organic liquid diluent suitable for use in the present process is typically an aliphatic or aromatic hydrocarbon, but can be any composition which will permit secondary alkyl sulfate precipitation or will permit phase separation from the glycol after extraction of the reaction mixture. As used herein, the term "nonionic organic liquid diluent" is used to refer to a diluent having characteristics such that the secondary alkyl sulfate product precipitates from solution whereas unreacted organic matter remain in solution. The nonionic organic liquid diluent, if inert to the specific step in the reaction sequence, may be added along with the detergent range olefins and/or alcohols at the beginning of the sulfation step, at any point during the sulfation step, after the sulfation reaction is completed, following the de-acidification step, or, it may be contacted with the alkyl sulfuric acid-containing solution during contact of the solution with the aqueous solution of base. Alternatively, the nonionic organic liquid diluent may be added at more than one of the aforementioned stages in the process, provided it is substantially inert during this process step. In a preferred embodiment, the nonionic organic liquid diluent is added to the alkyl sulfuric acid-containing solution following the sulfation step. Suitable nonionic organic liquid diluents include heptane, toluene, isooctane, nonane and mixtures thereof, with preference being given to heptane and isooctane, particularly heptane. Whilst not wishing to be bound by any particular theory, it is believed that the function of the nonionic organic liquid diluent in the present process is to provide a medium in which unreacted organic matter is soluble and secondary alkyl sulfate, at least in part, is not.

The crystallized secondary alkyl sulfate product of step d) is then recovered and dried as a solid surfactant composition as claimed in claim 10. The solid surfactant composition can be recovered by filtration or centrifugation. It is typically dried at temperatures in the range of from 40°C to 80°C using nitrogen-swept vacuum or other conventional drying means. Prior to drying, it may be subjected to washing with the particular nonionic organic liquid diluent used or with any other conventional washing agent in order to increase the purity thereof. In addition, it may be washed with ice-water.

The solid secondary alkyl sulfate-containing product produced in step d) contains at least 80 %wt to 99 %wt, preferably 85 %wt to 97 %wt of secondary alkyl sulfate, calculated on the weight of the solid recovered. The product generally contains some residual level of sodium sulfate. The product typically contains less than 20 %wt, preferably less than 10 %wt, sodium sulfate.

Following the removal and recovery of the crystallized solid surfactant composition in step d), the remainder of the product of step d) i.e., the uncrystallized portion of the reaction mixture, which contains unreacted starting material, secondary alcohol, nonionic organic liquid diluent, and some uncrystallized secondary alkyl sulfate, is contacted and mixed is subjected to step f) and g) as mentioned above. The uncrystallized portion of the product of step d) is, similar to the product of step c) in the second embodiment, contacted and mixed with a glycol in the presence of a nonionic organic liquid diluent.

The glycols suitable for use in step d or f) of the present process include alkyl glycols or polyether glycols of the formula 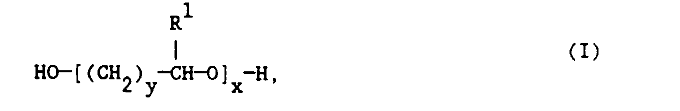 wherein x is an integer in the range of 1 to 20, y is an integer in the range of 1 to 3, and R¹ represents, individually in each occurrence, either hydrogen or methyl, with the proviso that R¹ is hydrogen when y is 2 or 3. The glycol molecules may contain from 1 to 10 ether groups, each of which may individually be ethoxy (-CH₂-CH₂-O-), propoxy (-CH₂-CH₂-CH₂-O-), or isopropoxy (-CH₂-CH(CH₃)-O-). The glycols may also, for convenience, be referred to as selected from the class consisting of C₂ and C₃ alkyl glycols, i.e., ethylene glycol, 1,2-propanediol, 1,3-propanediol, 1,4-propanediol, glycerine and also consisting of polyether glycol condensation products of between 2 and 20 of said alkyl glycols. Particularly good results are obtained using glycols of formula I wherein R¹ is hydrogen or methyl. Preferably, the glycol molecules contain from 1 to 10, more preferably from 1 to 8 ether groups. From the standpoint of availability and cost of the glycols, those with from 1 to 4 ether groups are still more preferred for use in the present invention.

Alkyl glycols and polyether glycols suitable for use in the present invention are well known and are commercially available materials. The lower glycols, e.g., ethylene glycol, diethylene glycol, tetraethylene glycol, propylene glycol, and 1,4-butanediol, etc., are specifically available, while the higher polyether glycols are typically available as mixtures of compounds having a range of ether groups. Specific or relatively narrow range higher glycols can be obtained from such mixtures, if desired, by distillation. In a particularly preferred embodiment, the glycol is an alkyl glycol or polyether glycol selected from the group consisting of 1,2-propanediol, diethylene glycol, ethylene glycol, 1,4-butanediol and mixtures thereof.

Following the contact and mixing of either the product of step c) (second embodiment) or the uncrystallized portion of the reaction mixture from step d) with a glycol in step f), the resulting mixture is phase separated in step g) into two phases, an upper phase and a lower phase. The upper phase contains primarily the nonionic organic liquid diluent (e.g., heptane), unreacted olefin and corresponding secondary alcohol. The lower phase contains primarily sodium secondary alkyl sulfate and glycol. The contacting and mixing and phase separating steps are dependent on the scale of operation and for small scale lab experiments are complete in a short time, e.g., < 15 minutes. Glycol extraction of the upper phase can be repeated as many times as is practical to attain increased recovery of sodium secondary alkyl sulfate from the upper phase that is rich in nonionic organic liquid diluent. Likewise, the lower phase can be washed with the nonionic organic liquid diluent to remove olefin and secondary alcohol if desired.

When suitable washing is complete, the concentration of the non-solid secondary alkyl sulfate/glycol-containing product can be adjusted by flashing excess glycol under vacuum and at a temperature which is generally less than 120 °C, depending on residence time at the elevated temperature. The amount of glycol removed from the lower phase depends to a large extent on the viscosity of the resulting non-solid secondary alkyl sulfate-containing composition and is generally limited to 50 %wt. The lower phase product which contains non-solid secondary alkyl sulfate and glycol typically contains from 5 %wt to 60 %wt secondary alkyl sulfate, calculated on the weight of the lower phase product.

The upper phase which contains unreacted starting material, secondary alcohol, and nonionic organic liquid diluent, as well as some residual amounts of secondary alkyl sulfate, may be recycled. If desired, the secondary alcohol can be separated from the unreacted starting material by means recognized by those skilled in the art such as, for example, distillation and/or crystallization. 2-Hexadecanol, for example, tends to crystallize on standing.

The process of the present invention may be carried out in a batch mode or in a continuous operation.

The solid and non-solid surfactant compositions prepared in accordance with the invention can be used in a variety of detergent applications. The solid surfactant compositions can be blended with solid detergent components such as, for example, sodium carbonate, in order to form dry detergent powders. The surfactant compositions can also be added to water or vice versa in order to form liquid detergents. The non-solid surfactant compositions can be adsorbed at relatively low temperature, 80 °C or less, onto solid detergent materials such as, for example, sodium carbonate, in order to form dry detergent powders. The non-solid surfactant compositions can also be used to formulate liquid detergent products.

To illustrate the invention, the following examples are provided.

### Example 1

To a 3-necked flask equipped with a paddle stirrer, thermometer, and addition flask topped with a nitrogen blanket was added 196.01 grams of NEODENE 14 (at least 95 %wt C₁₄ and at least 94 %wt normal alpha olefin) (NEODENE is a trademark of Shell Chemical Company). After cooling in an ice bath to 9 °C, 81.88 grams of 95 % sulfuric acid was added with good agitation at such a rate that the temperature did not exceed 19 °C. When the addition of sulfuric acid was complete, 80.00 grams of cold heptane was added, followed by the addition of 200.09 grams of 82 % sulfuric acid. After fifteen minutes of good agitation at 12-14°C, stirring was stopped and the mixture transferred to a separation funnel. When phase separation was complete at 20 °C, the lower phase was removed and weighed 212.25 grams. The upper phase, which weighed 345.00 grams, was titrated for acidity (1.166 meq/g (milliequivalents/gram)) and then added to 68.04 grams of 50 % sodium hydroxide and 60.09 grams of heptane in a 4-necked flask equipped with a paddle stirrer, thermometer, a Dean Stark trap with a nitrogen blanket, and an addition flask. The temperature in the reaction flask rose from about 24 °C to 70 °C during acid neutralization. When acid addition was complete, the addition flask was replaced with a nitrogen blanketed Dean Stark trap and the reactor heated to azeotrope water from the reaction mixture. Water began to azeotrope at a reactor temperature of about 95 °C. After 38.37 grams of water had distilled and the reaction temperature had risen to 106 °C, heating was discontinued and the heptane in the Dean Stark traps was returned to the reactor.

On cooling overnight to room temperature, the reactor contents were transferred to 2 centrifuge tubes with the aid of an additional 53.51 grams of heptane. The solid was separated from the mother liquor by centrifuging at 1500 revolutions per minute (rpm) for fifteen minutes at 25 °C. The mother liquor was decanted from the centrifuge tubes and the two solids were slurried with 80 grams of pentane each. Centrifugation was performed again as described above followed by mother liquor decantation and reslurry of the solid in pentane. This procedure was repeated two more times and then the solid product was transferred to a tarred dish and dried at 63 °C in a nitrogen-swept vacuum oven at 8 inches of mercury. The dried secondary tetradecyl sulfate weighed 145.63 grams. Analysis showed the recovered product to be 92.8 %wt anionic surfactant, 5.5 %wt sodium sulfate and 1.6 %wt sodium hydroxide. The product yield was 42.8 % mole (%m), calculated on the starting olefin. The results are presented in Table I.

### Example 2

Example 2 was carried out in a manner similar to Example 1 except that NEODENE 16 (tm, at least 92 %wt C₁₆ and at least 94 %wt normal alpha olefin) was used and toluene rather than heptane was used. Toluene (160 grams) was added after neutralization with 50 % sodium hydroxide. The results are presented in Table I.

### Example 3

Example 3 was carried out in a manner similar to Example 1 except that the sulfuric acid to NEODENE 16 (tm) ratio was 0.6 mole/mole, the 82 % acid wash was omitted and isooctane (208 grams) rather than heptane was used. The results are presented in Table I.

### Example 4

Example 4 was carried out in a manner similar to Example 1 except that a mixture of internal olefins (approximate composition: 4 %wt C₁₄; 47.6 %wt C₁₅; 34.0 %wt C₁₆; 11.9 %wt C₁₇ and 2.5 %wt C₁₈) was used as the olefin reactant. The internal olefins were distilled from NEODENE 1518 (tm, approximate distribution: 1.8 %wt C₁₄; 24.7 %wt C₁₅; 26.1 %wt C₁₆; 24.5 %wt C₁₇; 18.3 %wt C₁₈ and 4.6 %wt C₁₉). The results are presented in Table I.

### Example 5

To a resin kettle equipped with a paddle stirrer, thermometer, and addition flask topped with a nitrogen blanket was added 628.3 grams of NEODENE 14 (tm). After cooling to 14 °C, 197.5 grams of 95 % sulfuric acid was added with good agitation at such a rate that the temperature did not exceed 25 °C. When the addition of sulfuric acid was complete, 315 grams of heptane was added and the entire solution was then added to 185.5 grams of 50 % sodium hydroxide and 370.23 grams of distilled water in a 4-necked flask equipped with a paddle stirrer, thermometer, and Dean Stark traps with nitrogen blankets. The temperature in the reaction flask rose from about 34 °C to 68 °C during acid neutralization. When acid addition was complete, an additional 10.21 grams of 50 % sodium hydroxide and 945 grams of heptane were added to the reaction flask. The reactor was heated and water began to azeotrope at a reactor temperature of about 84 °C. After 471.15 grams of water had distilled and the reaction temperature had risen to 100 °C, heating was discontinued and the heptane in the Dean Stark traps was weighted and then discarded.

On cooling overnight to room temperature, the solid reaction product was recovered by filtration through two Buchner funnels fitted with Whatman #4 paper. The solid was washed with 3940 grams of heptane, after which the filter cakes were dried in a nitrogen-swept vacuum oven at 70 °C. The dried secondary tetradecyl sulfate weighed 456.14 grams. Analysis showed the recovered product to be 90.6 %wt anionic surfactant, 7.6 %wt sodium sulfate and 0.3 %wt sodium hydroxide. The product yield was 40.9 %m, calculated on the starting olefin. The results are presented in Table I.

### Example 6

Example 6 was carried out in a manner similar to Example 5 except that a mixture of internal olefins (as described in Example 4 above) and NEODENE 16 (tm, as described in Example 2 above) in a ratio of 60 %wt to 40 %wt, respectively, was used as the olefin reactant. The sulfation reaction was monitored until acidity was constant and then neutralization was performed in the usual manner. The results are presented in Table I.

### Example 7

Example 7 was carried out in a manner similar to Example 5 except that a mixture of 84.00 grams of NEODENE 16 (tm, as described in Example 2 above) and 30.26 grams of secondary hexadecanol (s-hexadecanol) (88 %wt 2-ol and 12 %wt 3-ol) was sulfated with 25.65 grams of 95 % sulfuric acid. When sulfation was complete, as determined by titration, the reaction sequence was completed as described in Example 5. The results are presented in Table I.

**Table I**

| | Olefin and/or Alcohol | Acid/Olefin and/or Alcohol (mole/mole) | | Secondary Alkyl Sulfate | |
|---|---|---|---|---|---|
| No. | Reactant | Ratio | Diluent | Recovered as solid (%m) | Purity (%wt) |
| 1 | NEODENE 14 | 0.8 | Heptane | 42.8 | 92.8 |
| 2 | NEODENE 16 | 0.8 | Toluene | 43.8 | 89.1 |
| 3 | NEODENE 16 | 0.6 | Isooctane | 44.8 | 82.4 |
| 4 | Internal | 0.8 | Heptane | 18.8 | 87.4 |
| | Olefin (C14/C18) | | | | |
| 5 | NEODENE 14 | 0.6 | Heptane | 40.9 | 90.6 |
| 6 | Internal | 0.6 | Heptane | 21.2 | 99 |
| | Olefin:NEODENE 16 (60:40) | | | | |
| 7 | NEODENE 16: | 0.5 | Heptane | 25.1 | 97.1 |
| | s-hexadecanol (74:26) | | | | |

### Example 8

To a 3-necked flask equipped with a paddle stirrer, thermometer, and addition flask topped with a nitrogen blanket was added 295.75 grams of NEODENE 16 (tm, as described in example 2 above) and 438.94 grams of C₁₄-C₁₈ internal olefin (3.5 %wt C₁₄; 41.8 %wt C₁₅; 36.5 %wt C₁₆; 15.3 %wt C₁₇; and 3.0 %wt C₁₈). After cooling in an ice bath to 17 °C, 203.95 grams of 95 % sulfuric acid was added with good agitation at such a rate that the temperature did not exceed 22 °C. When the addition of sulfuric acid was complete, acidity of the reaction mixture was monitored by titration until it remained essentially constant. The acidic reaction product was then added to a mixture of 203.88 grams of 50 % sodium hydroxide, 407.77 grams of distilled water and 1470 grams of heptane in a 4-necked flask equipped with a paddle stirrer, thermometer, a Dean Stark trap with a nitrogen blanket, and an addition flask. The temperature in the reaction flask rose to 48 °C during acid neutralization. When acid addition was complete, the addition flask was replaced with a nitrogen blanketed Dean Stark trap and the reactor heated to azeotrope water from the reaction mixture. Water began to azeotrope at a reactor temperature of 84 °C. After 525.28 grams of water had distilled and the reaction temperature had risen to 98 °C, heating was discontinued and the reaction mixture was cooled.

After cooling overnight to room temperature, the reactor contents were filtered through a Buchner funnel and washed with 4800 grams of heptane. The recovered solid weighed 232.75 grams after drying to constant weight in a vacuum oven at 70 °C. Mixed indicator titration for anionic showed the solid to be greater than 99 % pure sodium secondary alkyl sulfate.

A 200.00 gram aliquot was taken from the mother liquor from filtration and found to contain 55 meq. of dissolved sodium secondary alkyl sulfate. Extraction with 60.00 grams of propylene glycol followed by phase separation was found to transfer 31 meq. of sodium secondary alkyl sulfate to the propylene glycol phase. A second extraction of the mother liquor with 60.00 grams of propylene glycol transferred an additional 10 meq. of sodium secondary alkyl sulfate to the propylene glycol phase for an overall recovery of 74.5 % of non-solid sodium secondary alkyl sulfate that had been dissolved in the mother liquor.

### Example 9

To a 3-necked flask equipped with a paddle stirrer, thermometer, and addition flask topped with a nitrogen blanket was added 145.60 grams of NEODENE 16 (tm, as described in example 2 above). After cooling in an ice bath to 13 °C, 40.61 grams of 95 % sulfuric acid was added with good agitation at such a rate that the temperature did not exceed 26 °C. When the addition of sulfuric acid was complete, acidity of the reaction mixture was monitored by titration until it remained essentially constant. The acidic reaction product was then added to a mixture of 38.53 grams of 50 % sodium hydroxide, 215.00 grams of heptane, and 205.00 grams of propylene glycol in a 4-necked flask equipped with a paddle stirrer, thermometer, a Dean Stark trap with a nitrogen blanket, and an addition flask. The temperature in the reaction flask rose to 45 °C during acid neutralization. When acid addition was complete, the addition flask was replaced with a nitrogen blanketed Dean Stark trap and the reactor heated to azeotrope water from the reaction mixture. Water began to azeotrope at a reactor temperature of 92 °C. After 24.6 grams of water had distilled and the reaction temperature had risen to 100 °C, 102.23 grams of heptane was added to the reactor. After stirring for 28 minutes, stirring was discontinued and phase separation occurred in 5 minutes at 94 °C. The lower propylene glycol phase was a clear yellow colour, amounting to 378.24 grams and containing 296 meq. of anionic surfactant as determined by titration. The upper heptane phase weighed 235.33 grams and contained 8 meq. of anionic surfactant.

The propylene glycol phase was later concentrated using a rotovac distillation apparatus at 80-99 °C and a vacuum of 0.05-19.7 kPa (0.4-148 millimeters of Hg). The concentrated sodium hexadecyl sulfate in propylene glycol was a white paste containing 58 %wt of anionic surfactant.

### Example 10

To a 3-necked flask equipped with a paddle stirrer, thermometer, and addition flask topped with a nitrogen blanket was added 58.22 grams of NEODENE 16 (tm, as described in example 2 above) and 85.80 grams of C₁₄-C₁₈ internal olefin (as described in example 8 above). After cooling in an ice bath to 12 °C, 40.78 grams of 95 % sulfuric acid was added with good agitation at such a rate that the temperature did not exceed 24 °C. When the addition of sulfuric acid was complete, acidity of the reaction mixture was monitored by titration until it remained essentially constant. The acidic reaction product was then added to a mixture of 46.51 grams of 50 % sodium hydroxide, 465.10 grams of distilled water in a 4-necked flask equipped with a paddle stirrer, thermometer, condenser with a nitrogen blanket, and an addition flask. The temperature in the reaction flask rose to 41 °C during acid neutralization. When acid addition was complete, the addition flask was replaced with a glass stopper and the reactor heated to reflux. Samples were periodically removed from the reactor and analyzed for anionic content. When it held essentially constant, saponification was complete and 137.02 grams of propylene glycol was added to the reactor followed by 206.68 grams of heptane at a reactor temperature of 85-88 °C. After vigorous mixing, stirring was discontinued and phase separation occurred at a temperature in the range of 67-81 °C. The lower propylene glycol phase was clear, amounting to 787.63 grams and containing 276 meq. of anionic surfactant. The upper heptane phase weighed 184.47 grams and contained 1.3 meq. of anionic surfactant.

The propylene glycol phase was later concentrated using a rotovac distillation apparatus at 72-81 °C and a vacuum of 0.27-30 kPa (2-210 millimeters of Hg). The concentrated sodium hexadecyl sulfate in propylene glycol was a white paste containing 54 %wt of anionic surfactant.

## Claims

1. A process for preparing a surfactant composition comprising a secondary (C₈ to C₂₂) alkyl sulphate, which process comprises:
a) sulfating a (C₈ to C₂₂) olefin and/or a (C₈ to C₂₂) secondary alcohol, optionally containing a primary alcohol, with a sulfating agent,
b) neutralizing the sulfation product comprising alkyl sulfuric acid and dialkyl sulfate produced in step a) with a base in aqueous solution,
c) heating the product of step b) to saponify the dialkyl sulfate and to remove substantially all of the water from the mixture,
followed by either
d) cooling the product of step c) in the presence of a nonionic organic liquid diluent to ambient temperature whereby a crystallized solid composition separates, and
e) recovering and drying the crystallized solid composition of step d),
or by
f) contacting and mixing the product of step c) with a glycol in the presence of a nonionic organic liquid diluent,
g) phase separating the lower phase from the mixture formed in step f) which contains the glycol and the composition.

2. A process as claimed in claim 1, wherein the uncrystallized portion of the product of step d) is subjected to process steps f) and g).

3. A process as claimed in claim 1 or 2, wherein the recovered crystallized solid composition of step e) is washed with ice-water before a final drying step.

4. A process as claimed in claim 1, 2 or 3, wherein step d) is carried out in the presence of a glycol.

5. A process as claimed in any one of claims 1 to 4, wherein the glycol is selected from the group consisting of alkyl glycols, polyether glycols, glycerine and mixtures thereof.

6. A process as claimed in claim 5, wherein the glycol is selected from the group consisting of propylene glycol, ethylene glycol, diethylene glycol, and butylene glycol.

7. A process as claimed in any one of claims 1 to 6, wherein the nonionic organic liquid diluent is selected from the group consisting of aliphatic hydrocarbons, aromatic hydrocarbons and mixtures thereof.

8. A process as claimed in claim 7, wherein the nonionic organic liquid diluent is selected from the group consisting of heptane, toluene, isooctane, nonane and mixtures thereof.

9. A process as claimed in any one of claims 1 to 8, wherein the nonionic organic liquid diluent is added during step a), step b), and/or step c).

## Patentansprüche

1. Verfahren zur Herstellung einer Tensidzusammensetzung, die ein sek.- (C₈- bis C₂₂-)Alkylsulfat enthält, bei dem man:
a) ein (C₈- bis C₂₂-) Olefin und/oder einen (C₈- bis C₂₂-) sekundären Alkohol, der gegebenenfalls einen primären Alkohol enthält, mit einem Sulfatierungsmittel sulfatiert,
b) das in Schritt a) hergestellte, Alkylschwefelsäure und Dialkylsulfat enthaltende Sulfatierungsprodukt in wäßriger Lösung mit einer Base neutralisiert,
c) das Produkt aus Schritt b) zur Verseifung des Dialkylsulfats und zur weitgehend vollständigen Entfernung des Wassers aus der Mischung erhitzt
und anschließend entweder
d) das Produkt aus Schritt c) in Gegenwart eines nichtionischen organischen flüssigen Verdünnungsmittels auf Raumtemperatur abkühlt, wodurch sich eine kristallisierte feste Zusammensetzung abscheidet und
e) die kristallisierte feste Zusammensetzung aus Schritt d) isoliert und trocknet,
oder
f) das Produkt aus Schritt c) in Gegenwart eines nichtionischen organischen flüssigen Verdünnungsmittels mit einem Glykol in Berührung bringt und vermischt,
g) die untere Phase der in Schritt f) gebildeten Mischung, die das Glykol und die Zusammensetzung enthält, abtrennt.

2. Verfahren nach Anspruch 1, bei dem man den nichtkristallisierten Anteil des Produkts aus Schritt d) den Verfahrensschritten f) und g) unterwirft.

3. Verfahren nach Anspruch 1 oder 2, bei dem man die isolierte kristallisierte feste Zusammensetzung aus Schritt e) vor einem abschließenden Trocknungsschritt mit Eiswasser wäscht.

4. Verfahren nach Anspruch 1, 2 oder 3, bei dem man Schritt d) in Gegenwart eines Glykols durchführt.

5. Verfahren nach einem der Ansprüche 1 bis 4, bei dem man das Glykol aus der Gruppe bestehend aus Alkylglykolen, Polyetherglykolen, Glycerin und deren Gemischen auswählt.

6. Verfahren nach Anspruch 5, bei dem man das Glykol aus der Gruppe bestehend aus Propylenglykol, Ethylenglykol, Diethylenglykol und Butylenglykol auswählt.

7. Verfahren nach einem der Ansprüche 1 bis 6, bei dem man das nichtionische organische flüssige Verdünnungsmittel aus der Gruppe bestehend aus aliphatischen Kohlenwasserstoffen, aromatischen Kohlenwasserstoffen und deren Gemischen auswählt.

8. Verfahren nach Anspruch 7, bei dem man das nichtionische organische flüssige Verdünnungsmittel aus der Gruppe bestehend aus Heptan, Toluol, Isooctan, Nonan und deren Gemischen auswählt.

9. Verfahren nach einem der Ansprüche 1 bis 8, bei dem man das nichtionische organische flüssige Verdünnungsmittel während Schritt a), Schritt b) und/oder Schritt c) zusetzt.

## Revendications

1. Procédé de préparation d'une composition tensioactive ou surfactive comprenant un alkylsulfate (C₈ à C₂₂) secondaire, procédé qui comprend :
a) la sulfatation d'une oléfine en (C₈ à C₂₂) et/ou d'un alcool secondaire en (C₈ à C₂₂), contenant éventuellement un alcool primaire, avec un agent de sulfatation,
b) la neutralisation du produit de sulfatation comprenant de l'acide alkylsulfurique et un dialkylsulfate produit au cours de l'étape a) avec une base en solution aqueuse,
c) le chauffage du produit de l'étape b) pour saponifier le dialkylsulfate et pour sensiblement éliminer la totalité de l'eau du mélange,
opérations suivies
d) du refroidissement du produit de l'étape c) en présence d'un diluant liquide organique non ionique jusqu'à la température ambiante, si bien qu'une composition solide cristallisée se sépare, et
e) la récupération et le séchage de la composition solide cristallisée de l'étapes d),
ou bien
f) la mise en contact et le mélange du produit de l'étape c) avec un glycol en présence d'un diluant liquide organique non ionique,
g) la séparation des phases de la phase inférieure du mélange formé au cours de l'étape f), qui contient le glycol et la composition.

2. Procédé suivant la revendication 1, caractérisé en ce que la partie non cristallisée du produit de l'étape d) est soumise aux étapes opératoires f) et g).

3. Procédé suivant la revendication 1 ou 2, caractérisé en ce que la composition solide cristallisée de l'étape e) est lavée avec de l'eau glacée avant une étape de séchage finale.

4. Procédé suivant la revendication 1, 2 ou 3, caractérisé en ce que l'on entreprend l'étape d) en présence d'un glycol.

5. Procédé suivant l'une quelconque des revendications 1 à 4, caractérisé en ce que l'on choisit le glycol dans le groupe formé par les alkylglycols, les polyétherglycols, la glycérine et leurs mélanges.

6. Procédé suivant la revendication 5, caractérisé en ce que le glycol est choisi dans le groupe formé par le propylèneglycol, l'éthylèneglycol, le diéthylèneglycol et le butylèneglycol.

7. Procédé suivant l'une quelconque des revendications 1 à 6, caractérisé en ce que le diluant liquide organique non ionique est choisi dans le groupe formé par les hydrocarbures aliphatiques, les hydrocarbures aromatiques et leurs mélanges.

8. Procédé suivant la revendication 7, caractérisé en ce que le diluant liquide organique non ionique est choisi dans le groupe formé par l'heptane, le toluène, l'isooctane, le nonane et leurs mélanges.

9. Procédé suivant l'une quelconque des revendications 1 à 8, caractérisé en ce que le diluant liquide organique non ionique est ajouté au cours de l'étape a), de l'étape b) et/ou de l'étape c).
